Europäisches Patentamt

European Patent Office

Office européen des brevets

(19)

(11) Veröffentlichungsnummer: **0 244 560**
**B1**

(12) # EUROPÄISCHE PATENTSCHRIFT

(45) Veröffentlichungstag der Patentschrift:
06.12.89

(51) Int. Cl.⁴: **A61B 6/04**

(21) Anmeldenummer: 87101370.2

(22) Anmeldetag: 02.02.87

(54) **Röntgendiagnostikgerät mit einem Kipptisch.**

(30) Priorität: 05.05.86 DE 3615187

(43) Veröffentlichungstag der Anmeldung:
11.11.87 Patentblatt 87/46

(45) Bekanntmachung des Hinweises auf die Patenterteilung:
06.12.89 Patentblatt 89/49

(84) Benannte Vertragsstaaten:
DE FR

(56) Entgegenhaltungen:
EP-A- 0 146 006
US-A- 2 038 327
US-A- 3 379 877

(73) Patentinhaber: Siemens Aktiengesellschaft,
Wittelsbacherplatz 2, D-8000 München 2(DE)

(72) Erfinder: Brendl, Rudolf, Leipziger Strasse 49a,
D-8520 Erlangen(DE)
Erfinder: Hahn, Alfred, Dipl.-Ing. (FH), Bunsenstrasse 64,
D-8520 Erlangen(DE)
Erfinder: Weiss, Karl, Dipl.-Ing. (FH), Im Föhrenwald 23,
D-8521 Buckenhof(DE)

# Beschreibung

Die Erfindung betrifft ein Röntgendiagnostikgerät gemäß dem ersten Teil des Patentanspruches 1.

Ein Röntgendiagnostikgerät dieser Art ist in der EP-A-0 146 006 beschrieben. Es erlaubt bei geringem konstruktivem Aufwand eine Kippbewegung von beispielsweise 90° auf der einen Seite der horizontalen Lage und von 30° auf der anderen Seite. Die Führungsschienen sind bei diesem Röntgendiagnostikgerät alle geradlinig ausgebildet, so daß die Kippgeschwindigkeit bei konstanter Drehzahl des Kippmotors variiert.

Der Erfindung liegt die Aufgabe zugrunde, ein Röntgendiagnostikgerät der eingangs genannten Art so weiterzubilden, daß sich eine im wesentlichen gleichförmige Kippgeschwindigkeit ergibt.

Diese Aufgabe ist erfindungsgemäß gelöst durch den zweiten Teil des Patentanspruches 1. Bereits durch eine kreisförmige Krümmung um einen nahe dem Fußende der Tischplatte liegenden Punkt ist dabei eine etwa gleichförmige Kippgeschwindigkeit bei konstanter Drehzahl des Kippmotors erreichbar. Eine exakt gleichförmige Kippgeschwindigkeit kann durch eine entsprechende Kurvenform erzielt werden.

Die Erfindung ist nachfolgend anhand eines in der Zeichnung dargestellten Ausführungsbeispieles näher erläutert. Es zeigen:

Fig. 1 ein Röntgendiagnostikgerät nach der Erfindung in einer Seitenansicht, und

Fig. 2 und 3 das Röntgendiagnostikgerät gemäß Figur 1 in verschiedenen Kipplagen.

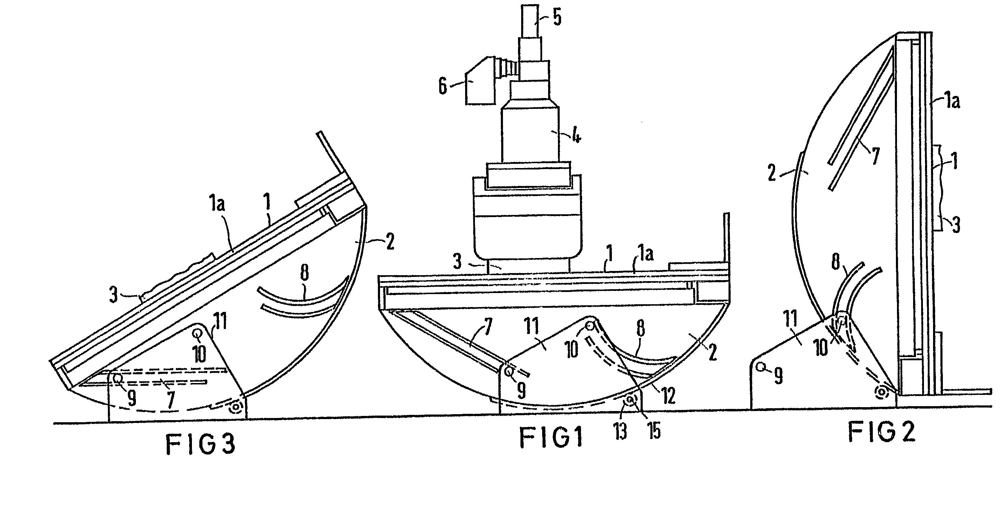

Das Gerät gemäß Figur 1 weist einen Kipptisch 1 auf, der auf einem Tischgestell 2 motorisch längsverschiebbar gelagert ist. Unterhalb des Kipptisches 1 liegt eine nicht dargestellte Röntgenröhre, die an einem Träger 3 befestigt ist, der oberhalb des Kipptisches 1 einen Röntgenbildverstärker 4 mit einer nachgeschalteten Fernsehkamera 5 und einer Blattfilmkamera 6 trägt. In der in der Figur 1 dargestellten Horizontallage des Kipptisches 1 liegt das Tischgestell 2 mit Führungsschienen, von denen in der Figur 1 die Führungsschienen 7, 8 sichtbar sind, auf Führungszapfen auf, von denen in der Figur 1 die Führungszapfen 9, 10 sichtbar sind. Die Führungszapfen 9, 10 sind an ortsfesten, seitlich am Tischgestell 2 angeordneten Lagerteilen befestigt, von denen in der Figur 1 das Lagerteil 11 sichtbar ist.

Das Tischgestell 2 ist an seiner Außenseite mit zwei kreisförmigen Antriebsbahnen versehen, von denen in der Figur 1 die Antriebsbahn 12 dargestellt ist. Mit der als Kette ausgebildeten Antriebsbahn 12 steht ein Zahnrad 13 in Eingriff, das durch einen Motor drehbar ist.

Zu den Teilen 7 bis 13 sind symmetrisch am Gerät nicht sichtbare, entsprechende Teile angeordnet, d.h., daß symmetrisch zu beiden Geräteseiten je eine Antriebsbahn entsprechend 12, ein Antriebsrad entsprechend 13, zwei Führungsschienen entsprechend 7, 8 und zwei Führungszapfen entsprechend 9, 10 vorhanden sind.

Soll der Kipptisch 1 aus seiner Horizontallage, in der sich das Tischgestell 2 mit den Führungsschienen 7, 8 auf den Führungszapfen 9, 10 abstützt, in eine vertikale Lage aufgerichtet werden, d.h. um 90° gedreht werden, so wird der Antriebsmotor eingeschaltet, der die Welle 15 und damit das Antriebsrad 13 dreht. Dabei bewegt sich das Tischgestell 2 mit dem Kipptisch 1 im Uhrzeigersinn und die Führungszapfen 9 verlassen die Führungsschienen 7. Bei dieser Kippbewegung ruht das Tischgestell 2 demgemäß dann noch mit den Führungsschienen 8 auf den Führungszapfen 10. Durch die Führung der Führungszapfen 10 in den Führungsschienen 8 wird erreicht, daß der Kipptisch 1 in die in der Figur 2 dargestellte Lage aufgerichtet werden kann, ohne daß er mit seinem unteren Ende am Boden anstößt.

Soll der Kipptisch 1 aus seiner in der Figur 1 dargestellten Horizontallage entgegen dem Uhrzeigersinn verschwenkt werden (Fig. 3), so verlassen in analoger Weise die Führungszapfen 10 die Führungsschienen 8 und die Führung erfolgt durch die Führungszapfen 9 in den Führungsschienen 7. Die Antriebsräder 13 drehen sich dabei entgegengesetzt zu ihrer Drehung bei der Aufrichtung in die Lage gemäß Figur 2. Das Tischgestell 2 wird dabei mit dem Kipptisch 1 entgegen dem Uhrzeigersinn in die in der Figur 3 dargestellte Kopf-Tief-Lage verschwenkt, in der Kipptisch 1 mit dem Boden einen Winkel von etwa 30° einschließt.

Beim Verstellen des Kipptisches 1 aus der Horizontallage gemäß Figur 1 in die Endlagen gemäß den Figuren 2 und 3 verläßt demgemäß jeweils einer der Führungszapfen 9, 10 seine Führungsbahn 7, 8 und das jeweils bodennähere Ende des Gerätes wird so weit angehoben, daß es über dem Bodenniveau liegt, wobei das Tischgestell 2 von den jeweiligen Führungszapfen 9, 10 in den Führungsschienen 7, 8 getragen wird.

Aus den Figuren 1 bis 3 ergibt sich, daß die nahe dem fußseitigen Ende der Tischplatte 1a liegenden Führungsschienen 8 um einen Punkt gekrümmt sind, der nahe diesem fußseitigen Ende liegt. Die Krümmung ist kreisbogenförmig. Dadurch ist erreicht, daß sich bei konstanter Drehzahl des Antriebsmotors eine etwa gleichförmige Kippgeschwindigkeit beim Kippen um 90° ergibt. Die Kippgeschwindigkeit ist dabei exakt gleichförmig, wenn eine geeignete Kurvenform der Führungsschienen 8 gewählt ist.

## Patentansprüche

1. Röntgendiagnostikgerät mit einem Kipptisch (1), der aus der horizontalen Lage nach beiden Seiten kippbar ist, davon nach einer Seite um 90°, bei dem der Kipptisch (1) auf einem Tischgestell (2) befestigt ist, das an seiner Außenseite mit kreisförmigen Antriebsbahnen (12) versehen ist, in die ein ortsfestes Antriebsrad (13) eingreift und bei dem am Tischgestell (2) auf jeder Seite zwei Führungsschienen (7, 8) angeordnet sind, in die in der Horizontallage des Tischgestelles (2) zwei ortsfeste Führungszapfen (9, 10) eingreifen und die derart ausgebildet und gerichtet sind, daß beim Verstellen des Kipptisches (1) aus der Horizontallage jeweils ein Führungszapfen (9, 10) seine Führungsschiene (7, 8) verläßt und der andere Führungszapfen über seine Führungsschiene eine derartige Verstellung des Tischgestelles (2) bewirkt, daß das untere Ende des Tischgestelles so weit angehoben wird, daß es über dem Bodenniveau liegt, wobei die dem Kopfende zugeordnet Führungsschiene (7) geradlinig ist, dadurch gekennzeichnet, daß die dem Fußende zugeordnete Führungsschiene (8) zur Tischplatte (1a) hin gekrümmt ist.

2. Röntgendiagnostikgerät nach Anspruch 1, **dadurch gekennzeichnet**, daß die gekrümmte Führungsschiene (8) um eine nahe dem Fußende der Tischplatte (1a) liegenden Punkt kreisbogenförmig gekrümmt ist.

## Claims

1. X-ray diagnostic apparatus having a tilting table (1) which can be tilted out of the horizontal position to both sides, thereby to one side by 90°, in which apparatus the tilting table (1) is secured to a table frame (2), which is provided on its outside with circular drive tracks (12) into which there engages a fixed drive wheel (13), and in which apparatus there are arranged on the table frame (2) on each side thereof two guide rails (7, 8) into which two fixed guide pins (9, 10) engage in the horizontal position of the table frame (2) and which are formed and directed such that upon adjustment of the tilting table (1) out of the horizontal position in each case one guide pin (9, 10) leaves its guide rail (7, 8) and the other guide pin, by way of its guide rail, effects such an adjustment of the table frame (2) that the lower end of the table frame is lifted to such an extend that it lies above the base level, the guide rail (7) associated with the head end being straight, characterised in that the guide rail (8) associated with the foot end is curved towards the table plate (1a).

2. X-ray diagnostic apparatus according to claim 1, characterised in that the curved guide rail (8) is curved in the form of an arc of a circle about a point lying close to the foot end of the table plate (1a).

## Revendications

1. Appareil de radiodiagnostic comportant une table basculante (1), qui peut basculer des deux côtés à partir de la position horizontale, et ce sur 90° d'un côté, et dans lequel la table basculante (1) est fixée sur un bâti (2) qui comporte, sur sa face extérieure, des pistes circulaires d'entraînement (12), dans lesquelles s'engage un pignon fixe d'entraînement (13), et dans lequel sur le bâti de la table se trouvent disposés, des deux côtes de ce bâti, deux glissières (7, 8), dans lesquelles s'engangent deux ergots fixes de guidage (9, 10), lorsque le bâti (2) de la table est horizontal, et qui sont agencées et orientées de telle sorte que, lors du déplacement de la table basculante (3) à partir de la position horizontale, respectivement un ergot de guidage (9, 10) quitte sa glissière (7, 8) et l'autre ergot de guidage provoque, le long de sa glissière, un déplacement tel du bâti (2) de la table que l'extrémité inférieure de ce bâti est soulevée au point qu'elle se situe au-dessus du niveau du sol, la glissière (7), associée à l'extrémité de tête, étant rectiligne, caractérisé par le fait que la glissière (8) associée à l'extrémité située du côté des pieds est cintrée en direction du plateau (1a) de la table.

2. Appareil de radiodiagnostic suivant la revendication 1, caractérisé par le fait que la glissière cintrée (8) est cintrée avec une forme en arc de cercle autour d'un point proche de l'extrémité, tournée du côté des pieds, du plateau (1a) de la table.

FIG 3

FIG 1

FIG 2

EP 0 244 560 B1